# EUROPÄISCHE PATENTSCHRIFT

(11) **EP 2 038 241 B1**
(45) Veröffentlichungstag und Bekanntmachung des Hinweises auf die Patenterteilung: **20.10.2010**
(21) Anmeldenummer: 07764773.3
(22) Anmeldetag: 22.06.2007
(51) Int. Cl.: C07C 17/35, C07C 25/22, C07C 211/54, C07C 211/56, C07D 221/18, C07D 311/78, C07D 311/80, C07D 409/14, C07F 9/6568, C08G 61/00, C08G 61/02, C08G 61/12, C09K 11/06, H01L 51/00, H05B 33/14

(54) **ELEKTROLUMINESZIERENDE POLYMERE UND IHRE VERWENDUNG**
ELECTROLUMINESCENT POLYMERS AND USE THEREOF
POLYMÈRES ÉLECTROLUMINESCENTS ET LEUR UTILISATION

(30) Priorität: 11.07.2006 DE 102006031991
(43) Veröffentlichungstag der Anmeldung: 25.03.2009
(73) Patentinhaber: Merck Patent GmbH, 64293 Darmstadt (DE)
(72) Erfinder: BUESING, Arne, 65929 Frankfurt am Main (DE); HEIL, Holger, 64295 Darmstadt (DE); LUDEMANN, Aurélie, 60322 Frankfurt (DE); SCHULTE, Niels, 65779 Kelkheim (DE)
(86) Internationale Anmeldenummer: PCT/EP2007/005500
(87) Internationale Veröffentlichungsnummer: WO 2008/006454

(56) Entgegenhaltungen:
- WO-A-98/39287
- WO-A-02/077060
- WO-A-03/020790
- WO-A1-01/42331
- WO-A1-2007/022845
- WO-A1-2007/022845
- DATABASE CA [Online] CHEMICAL ABSTRACTS SERVICE, COLUMBUS, OHIO, US; 18. Mai 2007 (2007-05-18), KOBAYASHI, SHIGEYA ET AL: "Conjugated polymeric compound and polymeric electroluminescence element using the same" XP002450929 gefunden im STN Database accession no. 2007:536920 -& WO 2007/055407 A (SUMITOMO CHEMICAL CO [JP]; KOBAYASHI SHIGEYA [JP]; KOBAYASHI SATOSHI [) 18. Mai 2007 (2007-05-18)

## Beschreibung

Die vorliegende Erfindung betrifft elektrolumineszierende Polymere, die Struktureinheiten der Formel (1) enthalten sowie deren Verwendung. Die erfindungsgemäßen Polymere zeigen eine verbesserte Effizienz und eine höhere Lebensdauer, insbesondere bei Verwendung in polymeren organischen Leuchtdioden.

Seit mehr als zehn Jahren läuft eine breit angelegte Forschung zur Kommerzialisierung von Anzeige- und Beleuchtungselementen auf Basis polymerer (organischer) Leuchtdioden (PLEDs). Ausgelöst wurde diese Entwicklung durch Grundlagenentwicklungen, welche in der WO 90/13148 A1 offenbart sind. Seit kurzem ist auch ein erstes, wenn auch einfaches, Produkt (eine kleine Anzeige in einem Rasierapparat der Fa. PHILIPS N.V.) auf dem Markt erhältlich. Allerdings sind immer noch deutliche Verbesserungen der verwendeten Materialien nötig, um diese Displays zu einer echten Konkurrenz zu den derzeit marktbeherrschenden Flüssigkristallanzeigen (LCD) zu machen.

Für die Erzeugung aller drei Emissionsfarben ist es notwendig, bestimmte Comonomere in die entsprechenden Polymere einzupolymerisieren (vgl. z.B. WO 00/046321 A1, WO 03/020790 A2 und WO 02/077060 A1). Auf diese Weise ist dann in der Regel, ausgehend von einem blau emittierenden Grundpolymer ("backbone"), die Erzeugung der beiden anderen Primärfarben Rot und Grün möglich.

Als Polymere für vollfarbige Anzeigeelemente (Full-Colour-Displays) wurden bereits verschiedene Materialklassen vorgeschlagen bzw. entwickelt. So kommen sowohl z.B. Poly-Fluoren-Derivate als auch Poly-Spirobifluoren-, Poly-Dihydrophenanthren- und Poly-IndenofluorenDerivate in Betracht. Auch Polymere, die eine Kombination der genannten Strukturelemente enthalten, wurden bereits vorgeschlagen. Darüber hinaus werden Polymere, welche Poly-para-phenylen (PPP) als Strukturelement enthalten, eingesetzt.

Die Polymere gemäß dem Stand der Technik zeigen teilweise schon gute Eigenschaften in der Anwendung in PLEDs. Trotz der bereits erzielten Fortschritte entsprechen diese Polymere allerdings noch nicht den Anforderungen, die an sie für hochwertige Anwendungen gestellt werden.

Insbesondere ist die Lebensdauer der grün und vor allem der blau emittierenden Polymere für viele Anwendungen noch nicht ausreichend. Gleiches gilt für die Effizienz der rot emittierenden Polymeren.

Es wurde nun überraschenderweise gefunden, dass eine neue Klasse von Polymeren sehr gute und den o.g. Stand der Technik übertreffende Eigenschaften aufweist. Diese Polymere und ihre Verwendung in PLEDs sind daher Gegenstand der vorliegenden Erfindung. Die neuen Struktureinheiten eignen sich insbesondere als Polymer-Grundgerüst, aber je nach Substitutionsmuster auch als Lochleiter, Elektronenleiter und/oder Emitter.

Gegenstand der Erfindung sind Polymere, enthaltend Einheiten der Formel (1), 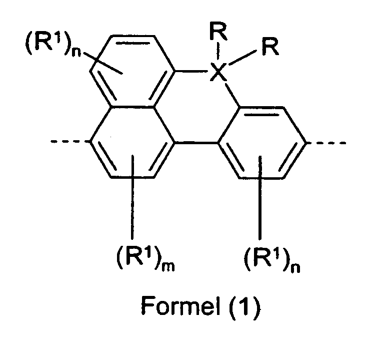 worin
- R: bei jedem Auftreten gleich oder verschieden H, eine geradkettige Alkylkette mit 1 bis 40 C-Atomen, eine verzweigte Alkylkette mit 3 bis 40 C-Atomen oder cyclische Alkylkette mit 3 bis 40 C-Atomen, die jeweils durch R¹ substituiert sein können, in der auch ein oder mehrere nicht benachbarte C-Atome durch =N-R¹, -O-, -S-, -O-CO-O-, -CO-O-, -CR¹=CR¹- oder -C≡C- ersetzt sein können und in der auch ein oder mehrere H-Atome durch F, Cl, Br, I oder CN ersetzt sein können, oder ein aromatisches Ringsystem mit 5 bis 40 C-Atomen oder hetero-aromatisches Ringsystem mit 2 bis 40 C- Atomen ist, welches auch durch ein oder mehrere Reste R¹ substituiert sein kann; dabei können die beiden Reste R miteinander auch ein weiteres mono- oder polycyclisches, aromatisches oder aliphatisches Ringsystem bilden; vorzugsweise mit der Maßgabe, dass mindestens einer oder beide Reste R ungleich H ist;
- R¹: bei jedem Auftreten gleich oder verschieden H, eine geradkettige Alkylkette mit 1 bis 22 C-Atomen, eine verzweigte Alkylkette mit 3 bis 22 C-Atomen oder cyclische Alkylkette mit 3 bis 22 C-Atomen, in der auch ein oder mehrere nicht benachbarte C-Atome durch =N-R², -O-, -S-, -O-CO-O-, -CO-O-, -CR²=CR²- oder -C≡C- ersetzt sein können und in der auch ein oder mehrere H-Atome durch F, Cl, Br, I oder CN ersetzt sein können, oder eine Aryl-, Heteroaryl-, Aryloxy- oder Heteroaryloxygruppe mit 5 bis 40 C-Atomen ist, welche auch durch ein oder mehrere nicht-aromatische Reste R¹ substituiert sein kann; dabei können auch zwei oder mehrere der Reste R¹ miteinander und/oder mit R ein Ringsystem bilden; oder F, Cl, Br, I, CN, N(R²)₂, Si(R²)₃ oder B(R²)₂ bedeuten;
- R²: bei jedem Auftreten gleich oder verschieden H oder ein aliphatischer oder aromatischer Kohlenwasserstoffrest mit 1 bis 20 C-Atomen ist;
- X: bei jedem Auftreten C ist;
- n: bei jedem Auftreten gleich oder verschieden 0, 1, 2 oder 3 ist;
- m: bei jedem Auftreten gleich oder verschieden 0, 1 oder 2 ist; und
die gestrichelte Bindung dabei in Formel (1) ebenso wie in allen weiteren Formeln die Verknüpfung im Polymer bedeutet. Sie soll hier keine Methylgruppe darstellen.

Bevorzugter Gegenstand der Erfindung sind Polymere, enthaltend Einheiten der Formel (1a), 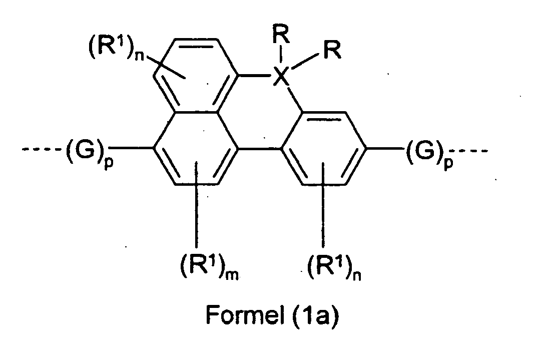 worin
- R: bei jedem Auftreten gleich oder verschieden H, eine geradkettige Alkylkette mit 1 bis 40 C-Atomen, eine verzweigte Alkylkette mit 3 bis 40 C-Atomen oder cyclische Alkylkette mit 3 bis 40 C-Atomen, die jeweils durch R¹ substituiert sein können, in der auch ein oder mehrere nicht benachbarte C-Atome durch =N-R¹, -O-, -S-, -O-CO-O-, -CO-O-, -CR¹=CR¹- oder -C≡C- ersetzt sein können und in der auch ein oder mehrere H-Atome durch F, Cl, Br, I oder CN ersetzt sein können, oder ein aromatisches Ringsystem mit 5 bis 40 C-Atomen oder hetero-aromatisches Ringsystem mit 2 bis 40 C- Atomen ist, welches auch durch ein oder mehrere Reste R¹ substituiert sein kann; dabei können die beiden Reste R miteinander auch ein weiteres mono- oder polycyclisches, aromatisches oder aliphatisches Ringsystem bilden; vorzugsweise mit der Maßgabe, dass mindestens einer oder beide Reste R ungleich H ist;
- R¹: bei jedem Auftreten gleich oder verschieden H, eine geradkettige Alkylkette mit 1 bis 22 C-Atomen, eine verzweigte Alkylkette mit 3 bis 22 C-Atomen oder cyclische Alkylkette mit 3 bis 22 C-Atomen, in der auch ein oder mehrere nicht benachbarte C-Atome durch =N-R², -O-, -S-, -O-CO-O-, -CO-O-, -CR²=CR²- oder -C≡C- ersetzt sein können und in der auch ein oder mehrere H-Atome durch F, Cl, Br, I oder CN ersetzt sein können, oder eine Aryl-, Heteroaryl-, Aryloxy- oder Heteroaryloxygruppe mit 5 bis 40 C-Atomen ist, welche auch durch ein oder mehrere nicht-aromatische Reste R¹ substituiert sein kann; dabei können auch zwei oder mehrere der Reste R¹ miteinander und/oder mit R ein Ringsystem bilden; oder F, Cl, Br, I, CN, N(R²)₂, Si(R²)₃ oder B(R²)₂ bedeuten;
- R²: bei jedem Auftreten gleich oder verschieden H oder ein aliphatischer oder aromatischer Kohlenwasserstoffrest mit 1 bis 20 C-Atomen ist;
- X: bei jedem Auftreten C ist;
- G: bei jedem Auftreten gleich oder verschieden ist und für eine bivalente Einheit ausgewählt aus der Gruppe 1, Gruppe 2, Gruppe 3, Gruppe 4, Gruppe 5, Gruppe 6, Gruppe 7 oder ein Gemisch aus mehreren der Gruppen 1 bis 7 steht;
- n: bei jedem Auftreten gleich oder verschieden 0, 1, 2 oder 3 ist;
- m: bei jedem Auftreten gleich oder verschieden 0, 1 oder 2 ist;
- p: bei jedem Auftreten gleich oder verschieden 0 oder 1 ist; und
die gestrichelte Bindung dabei in Formel (1a) ebenso wie in allen weiteren Formeln die Verknüpfung im Polymer bedeutet. Sie soll hier keine Methylgruppe darstellen.

Auch wenn dies aus der Beschreibung hervorgeht, sei hier nochmals explizit darauf verwiesen, dass die Struktureinheiten gemäß Formel (1) und (1a) unsymmetrisch substituiert sein können, d.h. dass an einer Einheit unterschiedliche Substituenten R bzw. R¹ vorhanden sein können.

Unter einem aromatischen bzw. heteroaromatischen Ringsystem im Sinne der vorliegenden Erfindung soll ein System verstanden werden, das nicht notwendigerweise nur aromatische bzw. heteroaromatische Gruppen enthält, sondern in dem auch mehrere aromatische bzw. heteroaromatische Gruppen durch eine kurze nicht-aromatische Einheit (< 10 % der von H verschiedenen Atome, vorzugsweise < 5 % der von H verschiedenen Atome), wie beispielsweise sp³-hybridisierter C, O, N, etc., unterbrochen sein können. So sollen also in der vorliegenden Anmeldung beispielsweise auch Systeme wie z.B. 9,9'-Spirobifluoren, 9,9-Diarylfluoren, Triarylamin, etc. als aromatische Ringsysteme verstanden werden.

Das erfindungsgemäße Polymer enthält üblicherweise mindestens 5 mol%, vorzugsweise mindestens 10 mol%, besonders bevorzugt mindestens 30 mol% und insbesondere mindestens 50 mol%, an Einheiten der Formel (1) bzw. (1a). Es hat sich gezeigt, dass wenn verschiedene Einheiten der Formel (1) bzw. (1a) gleichzeitig zugegen sind, die Summe aller Einheiten der Formel (1) bzw. (1a) im vorbezeichneten Bereich liegt, so dass der Gehalt an individuellen Einheiten der Formel (1) bzw. (1a) in solchen Fällen auch weniger als 5 mol% betragen kann.

Bei den erfindungsgemäßen Polymeren handelt es sich um konjugierte oder teilkonjugierte Polymere.

Konjugierte Polymere im Sinne der vorliegenden Anmeldung sind Polymere, die in der Hauptkette hauptsächlich sp²-hybridisierte Kohlenstoffatome, die auch durch entsprechende Heteroatome ersetzt sein können, enthalten. Dies bedeutet im einfachsten Fall abwechselndes Vorliegen von Doppel- und Einfachbindungen in der Hauptkette. Hauptsächlich meint, dass natürlich auftretende Defekte, die zu Konjugationsunterbrechungen führen, den Begriff "konjugiertes Polymer" nicht entwerten. Des Weiteren wird in diesem Anmeldungstext ebenfalls als konjugiert bezeichnet, wenn sich in der Hauptkette beispielsweise Arylamineinheiten und/oder bestimmte Heterocyclen (d.h. Konjugation über N-, O- oder S-Atome) und/oder metallorganische Komplexe (d.h. Konjugation über das Metallatom) befinden.

Hingegen werden Einheiten wie beispielsweise einfache Alkylbrücken, (Thio)Ether-, Ester-, Amid- oder Imidverknüpfungen eindeutig als nicht-konjugierte Segmente definiert. Unter einem teilkonjugierten Polymer soll ein Polymer verstanden werden, in dem längere konjugierte Abschnitte in der Hauptkette durch nicht-konjugierte Abschnitte unterbrochen sind, bzw. das längere konjugierte Abschnitte in den Seitenketten eines in der Hauptkette nicht-konjugierten Polymers enthält.

Die erfindungsgemäßen Polymere können neben den Einheiten gemäß Formel (1) bzw. (1a) noch weitere Strukturelemente enthalten. Dies sind u.a. solche, wie sie in der WO 02/077060 A1 und in der DE 10337346 A1 offenbart und umfangreich aufgelistet sind. Diese werden via Zitat als Bestandteil der vorliegenden Anmeldung betrachtet. Die weiteren Struktureinheiten können beispielsweise aus den folgenden Klassen stammen:

| | |
|---|---|
| Gruppe 1: | Einheiten, welche die Lochinjektions- und/oder -transporteigenschaften der Polymere erhöhen; |
| | |
| Gruppe 2: | Einheiten, welche die Elektroneninjektions- und/oder -transporteigenschaften der Polymere erhöhen; |
| | |
| Gruppe 3: | Einheiten, die Kombinationen von Einzeleinheiten der Gruppe 1 und Gruppe 2 aufweisen; |
| | |
| Gruppe 4: | Einheiten, welche die Emissionscharakteristik insoweit verändern, dass Elektrophosphoreszenz statt Elektrofluoreszenz erhalten werden kann; |
| | |
| Gruppe 5: | Einheiten, welche den Übergang vom so genannten Singulett- zum Triplettzustand verbessern; |
| | |
| Gruppe 6: | Einheiten, welche die Morphologie und/oder die Emissionsfarbe der resultierenden Polymere beeinflussen; |
| | |
| Gruppe 7: | Einheiten, welche typischerweise als Backbone verwendet werden. |

Bevorzugte erfindungsgemäße Polymere sind solche, bei denen mindestens ein Strukturelement Ladungstransporteigenschaften aufweist, d.h. die Einheiten aus den Gruppen 1 und/oder 2 enthalten.

Strukturelemente aus der Gruppe 1, die Lochtransporteigenschaften aufweisen, sind beispielsweise Triarylamin-, Benzidin-, Tetraaryl-para-phenylendiamin-, Triarylphosphin-, Phenothiazin-, Phenoxazin-, Dihydrophenazin-, Thianthren-, Dibenzo-para-dioxin-, Phenoxathiin-, Carbazol-, Azulen-, Thiophen-, Pyrrol- und Furanderivate und weitere O-, S- oder N-haltige Heterocyclen mit hoch liegendem HOMO (HOMO = höchstes besetztes Molekülorbital). Vorzugsweise führen diese Arylamine und Heterocyclen zu einem HOMO im Polymer von mehr als -5,8 eV (gegen Vakuumlevel), besonders bevorzugt von mehr als -5,5 eV.

Strukturelemente aus der Gruppe 2, die Elektronentransporteigenschaften aufweisen, sind beispielsweise Pyridin-, Pyrimidin-, Pyridazin-, Pyrazin-, Oxadiazol-, Chinolin-, Chinoxalin- und Phenazinderivate, aber auch Triarylborane und weitere O-, S- oder N-haltige Heterocyclen mit niedrig liegendem LUMO (LUMO = niedrigstes unbesetztes Molekülorbital). Vorzugsweise führen diese Einheiten im Polymer zu einem LUMO von weniger als -2,7 eV (gegen Vakuumlevel), besonders bevorzugt von weniger als -3,0 eV.

Es kann bevorzugt sein, wenn in den erfindungsgemäßen Polymeren Einheiten aus der Gruppe 3 enthalten sind, in denen Strukturen, welche die Lochmobilität und welche die Elektronenmobilität erhöhen (also Einheiten aus Gruppe 1 und 2), direkt aneinander gebunden sind. Einige dieser Einheiten können als Emitter dienen und verschieben die Emissionsfarbe ins Grüne, Gelbe oder Rote. Ihre Verwendung eignet sich also beispielsweise für die Erzeugung anderer Emissionsfarben aus ursprünglich blau emittierenden Polymeren.

Struktureinheiten gemäß der Gruppe 4 sind solche, welche auch bei Raumtemperatur mit hoher Effizienz aus dem Triplettzustand Licht emittieren können, also Elektrophosphoreszenz statt Elektrofluoreszenz zeigen, was häufig eine Steigerung der Energieeffizienz bewirkt. Hierfür eignen sich zunächst Verbindungen, welche Schweratome mit einer Ordnungszahl von mehr als 36 enthalten. Bevorzugt sind Verbindungen, welche d- oder f-Übergangsmetalle beinhalten, die die o.g. Bedingung erfüllen. Besonders bevorzugt sind hier entsprechende Struktureinheiten, welche Elemente der Gruppe 8 bis 10 (Ru, Os, Rh, Ir, Pd, Pt) enthalten. Als Struktureinheiten für die erfindungsgemäßen Polymeren kommen hier z.B. verschiedene Komplexe in Frage, wie sie z.B. in der WO 02/068435 A1, der DE 10116962 A1, der EP 1239526 A2 und der DE 10238903 A1 beschrieben werden. Entsprechende Monomere werden in der WO 02/068435 A1 und in der DE 10350606 A1 beschrieben.

Strukturelemente der Gruppe 5 sind solche, welche den Übergang vom Singulett- zum Triplettzustand verbessern und welche, unterstützend zu den Strukturelementen der Gruppe 4 eingesetzt, die Phosphoreszenzeigenschaften dieser Strukturelemente verbessern. Hierfür kommen insbesondere Carbazol- und überbrückte Carbazoldimereinheiten in Frage, wie sie in der DE 10304819 A1 und der DE 10328627 A1 beschrieben werden. Weiterhin kommen hierfür Ketone, Phosphinoxide, Sulfoxide und ähnliche Verbindungen in Frage, wie sie in der DE 10349033 A1 beschrieben werden.

Strukturelemente der Gruppe 6, die die Morphologie und/oder die Emissionsfarbe der Polymere beeinflussen, sind neben oben genannten solche, die mindestens noch eine weitere aromatische oder eine andere konjugierte Struktur aufweisen, welche nicht unter die o.g. Gruppen fallen, d.h. die die Ladungsträgermobilitäten nur wenig beeinflussen, die keine metallorganischen Komplexe sind oder die keinen Einfluss auf den Singulett-Triplett-Übergang haben. Derartige Strukturelemente können die Morphologie und/oder die Emissionsfarbe der resultierenden Polymere beeinflussen. Je nach Einheit können sie daher auch als Emitter eingesetzt werden. Bevorzugt sind dabei aromatische Strukturen mit 6 bis 40 C-Atomen oder auch Tolan-, Stilben- oder Bisstyrylarylenderivate, die jeweils mit einem oder mehreren Resten R¹ substituiert sein können. Besonders bevorzugt ist dabei der Einbau von 1,4 Phenylen-, 1,4-Naphthylen-, 1,4- oder 9,10-Anthrylen-, 1,6-, 2,7- oder 4,9-Pyrenylen-, 3,9- oder 3,10- Perylenylen-, 4,4'-Biphenylylen-, 4,4" Terphenylylen, 4,4'-Bi-1,1'-naphthylylen-, 4,4'-Tolanylen-, 4,4'-Stilbenylen- oder 4,4" Bisstyrylarylenderivaten.

Strukturelemente der Gruppe 7 sind Einheiten, die aromatische Strukturen mit 6 bis 40 C-Atomen beinhalten, welche typischerweise als Polymergrundgerüst (Backbone) verwendet werden. Dies sind beispielsweise 4,5 Dihydropyrenderivate, 4,5,9,10-Tetrahydropyrenderivate, Fluorenderivate, 9,9'-Spirobifluorenderivate, 9,10-Dihydrophenanthrenderivate, 5,7-Dihydrodibenzooxepinderivate und cis- und trans-Indenofluorenderivate. Da jedoch der Anteil an Einheiten gemäß Formel (1) bzw. (1a) insbesondere mindestens 50 mol% beträgt, werden diese Strukturelemente aus Gruppe 7 hier nicht vorzugsweise als das hauptsächliche Polymergrundgerüst, sondern höchstens als Grundgerüst, das in geringerem Anteil anwesend ist, eingesetzt.

Bevorzugt sind erfindungsgemäße Polymere, die gleichzeitig neben Struktureinheiten der Formel (1) bzw. (1a) zusätzlich noch ein oder mehrere Einheiten ausgewählt aus den Gruppen 1 bis 7 enthalten. Es kann ebenfalls bevorzugt sein, wenn gleichzeitig mehr als eine Struktureinheit aus einer Gruppe vorliegt.

Vorzugsweise beträgt der Anteil an Einheiten der Formel (1) bzw. (1a) mindestens 10 mol%, besonders bevorzugt mindestens 30 mol% und insbesondere mindestens 50 mol%. Diese Bevorzugung gilt vor allem, wenn es sich bei den Einheiten der Formel (1) bzw. (1a) um das Polymer-Grundgerüst handelt. Bei anderen Funktionen können andere Anteile bevorzugt sein, beispielsweise ein Anteil in der Größenordnung von 5 bis 20 mol%, wenn es sich um den Lochleiter bzw. den Emitter in einem elektrolumineszierenden Polymer handelt. Für andere Anwendungen, beispielsweise für organische Transistoren, kann der bevorzugte Anteil nochmals unterschiedlich sein, beispielsweise bis zu 100 mol%, wenn es sich um loch- oder elektronenleitende Einheiten handelt.

Bevorzugt sind erfindungsgemäße Polymere, die außer Struktureinheiten der Formel (1) bzw. (1a) noch mindestens eine Struktureinheit aus den oben genannten Gruppen enthalten. Besonders bevorzugt sind mindestens zwei Struktureinheiten aus unterschiedlichen der oben genannten Klassen. Wenn vorhanden, ist der Anteil dieser Strukturelemente vorzugsweise jeweils mindestens 5 mol%, besonders bevorzugt jeweils mindestens 10 mol%. Insbesondere ist eine dieser Struktureinheiten aus der Gruppe der lochleitenden Einheiten ausgewählt und die andere Gruppe ist eine emittierende Einheit, wobei diese beiden Funktionen (Lochleitung und Emission) auch von derselben Einheit übernommen werden können.

Aber auch ein kleinerer Anteil der emittierenden Einheiten, insbesondere grün und rot emittierender Einheiten, kann bevorzugt sein, beispielsweise zur Synthese von weiß emittierenden Copolymeren. Wie weiß emittierende Copolymere synthetisiert werden können, ist im Detail in der DE 10343606 A1 beschrieben.

Die erfindungsgemäßen Polymere weisen vorzugsweise 10 bis 10000, besonders bevorzugt 50 bis 5000 und insbesondere 50 bis 2000 Wiederholeinheiten auf.

Die nötige Löslichkeit der Polymere wird u.a. durch die Substituenten R bzw. R¹ an den Einheiten der Formel (1) bzw. (1a) sowie gegebenenfalls an weiteren anwesenden Einheiten gewährleistet. Falls weitere Substituenten vorhanden sind, können auch diese zur Löslichkeit beitragen.

Um eine ausreichende Löslichkeit zu gewährleisten, ist es bevorzugt, dass im Durchschnitt pro Wiederholeinheit mindestens 2 nicht-aromatische C-Atome in den Substituenten vorhanden sind. Vorzugsweise sind dabei mindestens 4 und besonders bevorzugt mindestens 8 C-Atome. Einzelne dieser C-Atome können auch durch O oder S ersetzt sein. Dies kann aber durchaus bedeuten, dass ein gewisser Anteil von Wiederholeinheiten keine weiteren nicht-aromatischen Substituenten trägt.

Um die Morphologie des Films nicht zu verschlechtern, ist es bevorzugt, keine langkettigen Substituenten mit mehr als 12 C-Atomen in einer linearen Kette zu haben, besonders bevorzugt keine mit mehr als 8 C-Atomen und insbesondere keine mit mehr als 6 C-Atomen.

Nicht-aromatische C-Atome sind, wie beispielsweise in der Beschreibung für R und R¹ in Formel (1) bzw. (1a), in entsprechenden geradkettigen, verzweigten oder cyclischen Alkyl- oder Alkoxyketten enthalten.

Bevorzugt sind erfindungsgemäße Polymere, bei denen für Einheiten der Formel (1) bzw. (1a) gilt, dass
- R: bei jedem Auftreten gleich oder verschieden eine geradkettige Alkylkette mit 1 bis 25 C-Atomen, eine verzweigte Alkylkette mit 3 bis 25 C-Atomen oder cyclische Alkylkette mit 3 bis 25 C-Atomen, in der auch ein oder mehrere nicht benachbarte C-Atome durch =N-R¹, -O-, -S-, -O-CO-O-, -CO-O-, -CH=CH- oder -C≡C- ersetzt sein können und in der auch ein oder mehrere H-Atome durch F oder CN ersetzt sein können, oder ein aromatisches Ringsystem mit 5 bis 20 C- Atomen oder hetero-aromatisches Ringsystem mit 2 bis 20 C-Atomen ist, welches auch durch ein oder mehrere nicht-aromatische Reste R¹ substituiert sein kann; dabei können die beiden Reste R miteinander auch ein weiteres mono- oder polycyclisches, aromatisches oder aliphatisches Ringsystem bilden;
- R¹: bei jedem Auftreten gleich oder verschieden H, eine geradkettige Alkylkette mit 1 bis 22 C-Atomen, eine verzweigte Alkylkette mit 3 bis 22 C-Atomen oder cyclische Alkylkette mit 3 bis 22 C-Atomen, in der auch ein oder mehrere nicht benachbarte C-Atome durch =N-R², -O-, -S-, -O-CO-O-, -CO-O-, -CH=CH- oder -C≡C- ersetzt sein können und in der auch ein oder mehrere H-Atome durch F oder CN ersetzt sein können, oder eine Aryl-, Heteroaryl-, Aryloxy- oder Heteroaryloxy- gruppe mit 5 bis 40 C-Atomen ist, welche auch durch ein oder mehrere nicht-aromatische Reste R¹ substituiert sein kann; dabei können auch zwei oder mehrere der Reste R¹ miteinander und/oder mit R ein mono- oder polycyclisches, aliphatisches oder aromatisches Ringsystem bilden; oder F, Cl, Br, I, CN, N(R²)₂, Si(R²)₃ oder B(R²)₂ bedeuten; und
- X: bei jedem Auftreten C ist.

Besonders bevorzugt sind erfindungsgemäße Polymere, bei denen für Einheiten der Formel (1) bzw. (1a) gilt, dass
- R: bei jedem Auftreten gleich oder verschieden eine geradkettige, verzweigte oder cyclische Alkylkette mit 4 bis 20 C-Atomen, besonders bevorzugt eine verzweigte Alkylkette ist, in der auch ein oder mehrere nicht benachbarte C-Atome durch =N-R¹, -O-, -S-, -O-CO-O-, -CO-O-, -CH=CH- oder -C≡C- ersetzt sein können und in der auch ein oder mehrere H-Atome durch F ersetzt sein können; dabei können die beiden Reste R miteinander auch ein weiteres mono- oder polycyclisches Ringsystem bilden;
- X: bei jedem Auftreten C ist; und
- p: für die Zahl Null steht.

Die Bevorzugung aliphatischer Reste R lässt sich durch die nochmals bessere Löslichkeit der resultierenden Polymere und die bessere synthetische Zugänglichkeit begründen.

Je nach Substitutionsmuster eignen sich die Einheiten der Formel (1) bzw. (1a) insbesondere für verschiedene Funktionen im Polymer. So können diese Einheiten vorzugsweise als (elektronenleitendes) Polymer-Grundgerüst, als Lochleiter oder als Emitter eingesetzt werden.

Weiterhin bevorzugt sind Einheiten der Formel (1) bzw. (1a), die in den 7,7'-Positionen symmetrisch substituiert sind. Diese Bevorzugung ist durch die bessere synthetische Zugänglichkeit der Monomere zu begründen. Vorzugsweise gilt also, dass in einer Einheit der Formel (1) bzw. (1a) alle R gleich und besonders bevorzugt auch gleich substituiert sind.

Beispiele für bevorzugte Einheiten der Formel (1) bzw. (1a) sind die folgenden Strukturen wobei die Verknüpfung im Polymer jeweils über die 3,9-Positionen erfolgt, wie über die gestrichelten Bindungen angedeutet. Mögliche Substituenten sind wegen der Übersichtlichkeit im Allgemeinen nicht bzw. nicht überall aufgeführt. Alkyl steht hier allgemein für eine aliphatische Alkylgruppe, Aryl für ein aromatisches oder heteroaromatisches System, wie für R beschrieben.

| | |
|---|---|
| 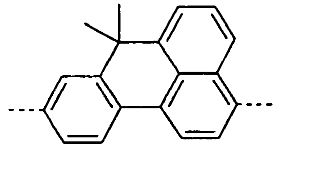 | 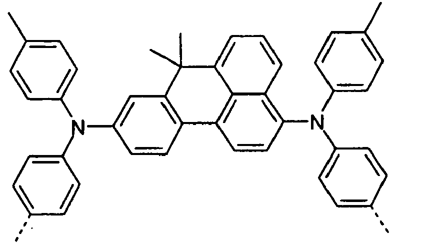 |
| Formel 1 | Formel 2 |
| 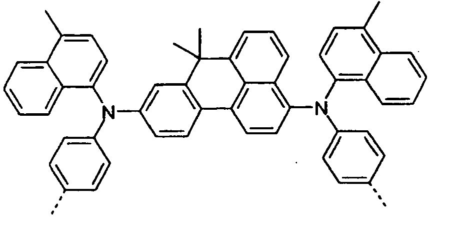 | 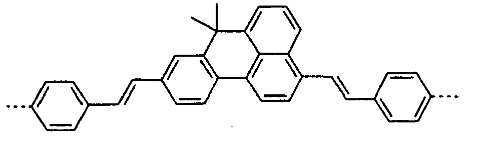 |
| Formel 3 | Formel 4 |
| 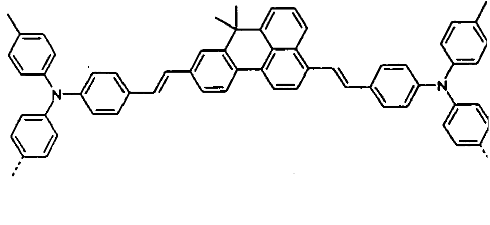 | 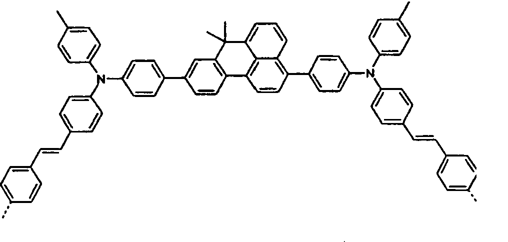 |
| Formel 5 | Formel 6 |
| 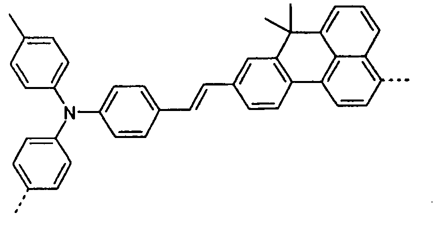 | 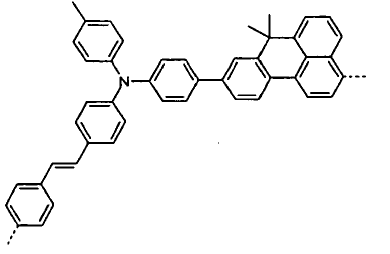 |
| Formel 7 | Formel 8 |
| 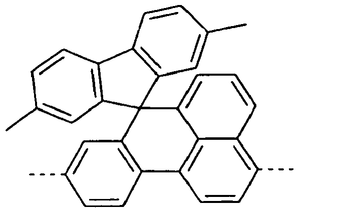 | |
| Formel 9 | |

Die erfindungsgemäßen Polymere sind entweder Homopolymere oder Copolymere. Erfindungsgemäße Copolymere können dabei neben einer oder mehreren Strukturen der Formel (1) bzw. (1a) potenziell eine oder mehrere weitere Strukturen, beispielsweise aus den oben genannten Gruppen 1 bis 7, aufweisen.

Die erfindungsgemäßen Copolymere können statistische, alternierende oder blockartige Strukturen aufweisen oder auch mehrere dieser Strukturen abwechselnd besitzen. Wie Copolymere mit blockartigen Strukturen erhalten werden können, ist beispielsweise ausführlich in der DE 10337077 A1 beschrieben. Diese Offenlegungsschrift ist via Zitat Bestandteil der vorliegenden Anmeldung.

Durch das Verwenden mehrerer verschiedener Strukturelemente können Eigenschaften wie Löslichkeit, Festphasenmorphologie, Farbe, Ladungsinjektions- und -transporteigenschaften, Temperaturstabilität, elektrooptische Charakteristik etc. eingestellt werden.

Die erfindungsgemäßen Polymere werden in der Regel durch Polymerisation von einer oder mehreren Monomersorten hergestellt, von denen mindestens ein Monomer im Polymer zu Einheiten der Formel (1) bzw. (1a) führt. Entsprechende Polymerisationsreaktionen gibt es prinzipiell viele. Es haben sich hier jedoch einige Typen besonders bewährt, die zu C-C- bzw. zu C-N-Verknüpfungen führen:
(A) Polymerisation gemäß SUZUKI;
(B) Polymerisation gemäß YAMAMOTO;
(C) Polymerisation gemäß STILLE;
(D) Polymerisation gemäß HARTWIG-BUCHWALD.

Wie die Polymerisation nach diesen Methoden durchgeführt werden kann und wie die Polymere vom Reaktionsmedium abgetrennt und aufgereinigt werden können, ist beispielsweise im Detail in der DE 10249723 A1 beschrieben.

Monomere, die in den erfindungsgemäßen Polymeren zu Struktureinheiten der Formel (1) führen, sind Derivate, die an der 3,9-Position geeignete Funktionalitäten aufweisen, die es erlauben, diese Monomereinheit in das Polymer einzubauen. Insofern eine andere Verknüpfung gewünscht ist, ist die Funktionalität entsprechend zu ändern.

Monomere, die im Polymer zu Einheiten der Formel (1) bzw. (1a) führen, sind bifunktionelle monomere Verbindungen der Formel (2) bzw. (2a), 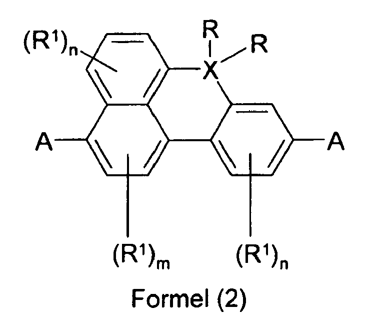 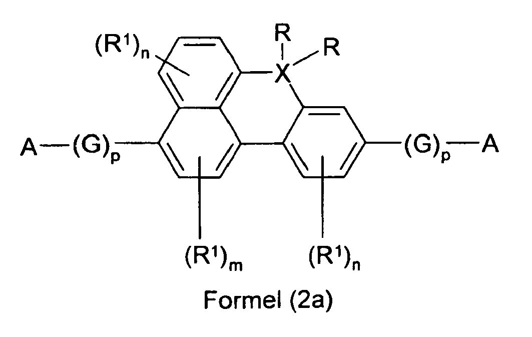 die dadurch gekennzeichnet sind, dass die beiden funktionellen Gruppen A, gleich oder verschieden, unter Bedingungen der C-C- bzw. C-N-Verknüpfungen copolymerisieren, wobei die weiteren Symbole und Indices dieselbe Bedeutung wie in bezug auf Formel (1) bzw. (1a) haben.

Vorzugsweise ist A ausgewählt aus Cl, Br, I, O-Tosylat, O-Triflat, O-SO₂R², B(OR²)₂ und Sn(R²)₃, besonders bevorzugt aus Br, I und B(OR²)₂, wobei R² dieselbe Bedeutung hat, wie oben beschrieben, und wobei zwei oder mehrere Reste R² auch miteinander ein Ringsystem bilden können.

Die C-C-Verknüpfungen sind vorzugsweise ausgewählt aus den Gruppen der SUZUKI-Kupplung, der YAMAMOTO-Kupplung und der STILLE-Kupplung; die C-N-Verknüpfung ist vorzugsweise eine Kupplung gemäß HARTWIG-BUCHWALD.

Dabei gilt für bifunktionelle monomere Verbindungen der Formel (2) bzw. (2a) dieselbe Bevorzugung wie sie für die Struktureinheiten der Formel (1) bzw. (1a) oben beschrieben ist.

Es kann bevorzugt sein, das erfindungsgemäße Polymer nicht als Reinsubstanz, sondern als Mischung (Blend) zusammen mit weiteren beliebigen polymeren, oligomeren, dendritischen oder niedermolekularen Substanzen zu verwenden. Diese können beispielsweise die elektronischen Eigenschaften verbessern, den Transfer vom Singulett- zum Triplettzustand beeinflussen oder selber aus dem Singulett- oder aus dem Triplettzustand Licht emittieren. Aber auch elektronisch inerte Substanzen können sinnvoll sein, um beispielsweise die Morphologie des gebildeten Polymerfilms oder die Viskosität von Polymerlösungen zu beeinflussen. Solche Blends sind daher auch Gegenstand der vorliegenden Erfindung.

Gegenstand der Erfindung sind weiterhin Lösungen und Formulierungen aus einem oder mehreren erfindungsgemäßen Polymeren oder Blends in einem oder mehreren Lösungsmitteln. Wie Polymerlösungen hergestellt werden können, ist beispielsweise in der WO 02/072714 A1, in der WO 03/019694 A2 und in der darin zitierten Literatur beschrieben. Diese Lösungen können verwendet werden, um dünne Polymerschichten herzustellen, zum Beispiel durch Flächenbeschichtungsverfahren (z.B. Spin-coating) oder Druckverfahren (z.B. Ink Jet Printing).

Die erfindungsgemäßen Polymere können in PLEDs verwendet werden. Diese enthalten Kathode, Anode, Emissionsschicht und gegebenenfalls weitere Schichten, wie z.B. vorzugsweise eine Lochinjektionsschicht und gegebenenfalls eine Zwischenschicht zwischen der Lochinjektions- und der Emissionsschicht. Wie PLEDs hergestellt werden können, wird als allgemeines Verfahren ausführlich in der DE 10304819 A1 beschrieben, das entsprechend für den Einzelfall anzupassen ist.

Wie oben beschrieben, eignen sich die erfindungsgemäßen Polymere ganz besonders als Elektrolumineszenzmaterialien in den derart hergestellten PLEDs oder Displays.

Als Elektrolumineszenzmaterialien im Sinne der Erfindung gelten Materialien, die als aktive Schicht in einer PLED Verwendung finden können. Aktive Schicht bedeutet, dass die Schicht befähigt ist, bei Anlegen eines elektrischen Feldes Licht abzustrahlen (lichtemittierende Schicht) und/oder dass sie die Injektion und/oder den Transport der positiven und/oder negativen Ladungen verbessert (Ladungsinjektions- oder Ladungstransportschicht). Es kann sich auch um eine Zwischenschicht zwischen einer Lochinjektionsschicht und einer Emissionsschicht handeln.

Gegenstand der Erfindung ist daher auch die Verwendung eines erfindungsgemäßen Polymers in einer PLED, insbesondere als Elektrolumineszenzmaterial.

Gegenstand der Erfindung ist somit ebenfalls eine PLED mit einer oder mehreren aktiven Schichten, wobei mindestens eine dieser aktiven Schichten ein oder mehrere erfindungsgemäße Polymere enthält. Die aktive Schicht kann beispielsweise eine lichtemittierende Schicht und/oder eine Transportschicht und/oder eine Ladungsinjektionsschicht und/oder eine Zwischenschicht sein.

Die erfindungsgemäßen Polymere weisen gegenüber den in der WO 03/020790 A2 und der WO 02/077060 A1 beschriebenen Poly-Spirobifluorenen und Polyfluorenen, die hiermit als nächstliegender Stand der Technik genannt werden, folgende überraschende Vorteile auf:
(1) Es wurde gefunden, dass die erfindungsgemäßen Polymere (bei ansonsten gleicher oder ähnlicher Zusammensetzung) höhere Leuchteffizienzen in der Anwendung aufweisen. Dies gilt besonders für die Copolymere, die blaue Emission zeigen. Dies ist von enormer Bedeutung, da somit entweder gleiche Helligkeit bei geringerem Energieverbrauch erzielt werden kann, was vor allem bei mobilen Applikationen (Displays für Handys, Pager, PDA etc.), die auf Batterien und Akkus angewiesen sind, sehr wichtig ist. Umgekehrt erhält man bei gleichem Energieverbrauch höhere Helligkeiten, was beispielsweise für Beleuchtungsanwendungen interessant sein kann.
(2) Des Weiteren hat sich überraschenderweise gezeigt, dass wiederum im direkten Vergleich die erfindungsgemäßen Polymere höhere operative Lebensdauern aufweisen, insbesondere im Fall von grün und blau emittierenden PLEDs.
(3) Die erfindungsgemäßen Polymere sind, auch ohne den Einsatz von elektronenleitenden Comonomeren, gute Elektronenleiter. Elektronenleitende Eigenschaften in Polymeren sind bislang schwierig zu verwirklichen gewesen, da viele Elektronenleiter gemäß dem Stand der Technik für hochwertige Anwendungen nicht ausreichend stabil sind.

Im vorliegenden Anmeldungstext und auch in den im Weiteren folgenden Beispielen wird auf die Verwendung erfindungsgemäßer Polymere oder Blends in Bezug auf PLEDs und die entsprechenden Displays abgezielt. Trotz dieser Beschränkung der Beschreibung ist es für den Fachmann ohne weiteres erfinderisches Zutun möglich, die erfindungsgemäßen Polymere auch für weitere Verwendungen in anderen elektronischen Devices (Vorrichtungen) zu benutzen, z.B. für organische integrierte Schaltungen (O-ICs), organische Feld-Effekt-Transistoren (OFETs), organische Dünnfilmtransistoren (OTFTs), organische Solarzellen (O-SCs) oder auch organische Laserdioden (O-Laser), um nur einige Anwendungen zu nennen.

Die Verwendung erfindungsgemäßer Polymere in den entsprechenden Vorrichtungen ebenso wie diese Vorrichtungen selbst sind ebenfalls Gegenstand der vorliegenden Anmeldung.

Die vorliegende Erfindung wird im Folgenden anhand von Ausführungsbeispielen näher erläutert, ohne dadurch jedoch eingeschränkt zu werden.

### Beispiele:

### Beispiel 1: Herstellung von 3,9-Dibrom-7,7-dimethyl-7H-benzofdelanthracen

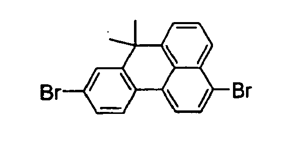

### 1.1 Herstellung von 3,9-Dibrom-benzo[de]anthracen-7-on

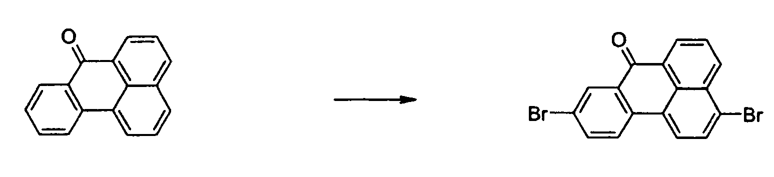

50 g (217 mmol) Benzanthron werden in 800 ml Nitrobenzol vorgelegt und auf eine Innentemperatur von 90°C gebracht. 25 ml (490 mmol) Brom in 100 ml Nitrobenzol gelöst werden innerhalb 30 Minuten zugetropft. Danach wird das Reaktionsgemisch auf eine Innentemperatur von 150°C erhitzt. Nach 2 Stunden wird auf Raumtemperatur abgekühlt, 200 ml EtOH zugetropft, der gelbe Niederschlag abgesaugt, mit viel EtOH gewaschen und getrocknet. Man erhält 49,7 g (59%) des dibromierten Produktes als gelbes Pulver.

### 1.2 Herstellung von 3,9-Dibrom-7H-benzo[de]anthracen

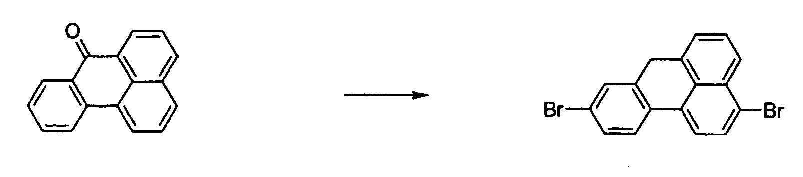

In einer ausgeheizten Apparatur werden 37,17 g (81,7 mmol) Dibrombenzanthron und 10,9 g (81,7 mmol) AlCl₃ unter N₂ in 330 ml getrocknetem Diethylether vorgelegt. Dann werden unter Eiskühlung 3,1 g (81,7 mmol) LiAlH₄ in 400 ml Ether (vorher unter Eiskühlung unter N₂ gelöst bzw. suspendiert) zugetropft. Nach beendeter Zugabe wird noch 60 Minuten zum Sieden erhitzt.

Es werden unter Eiskühlung langsam 10 ml Essigsäureethylester zugegeben, anschließend vorsichtig 100 ml 6 M HCl zugetropft und der entstandene Niederschlag abgesaugt, mit 1 M HCl und MeOH gewaschen und trockengesaugt.

Man erhält 25,6 g (84%) eines hellgelben Feststoffes.

### 1.3 Herstellung von 3,9-Dibrom-7,7-dimethyl-7H-benzo[de]anthracen

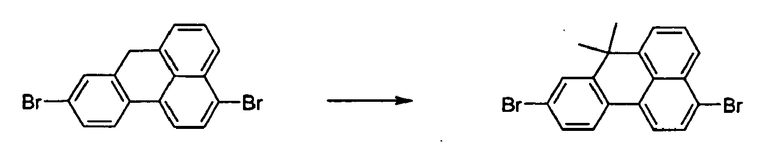

26 g (69 mmol) 3,9-Dibrom-7H-benzo[de]anthracen werden in 280 ml getrocknetem DMSO bei 70°C gelöst, 40 g (416 mmol) NaO*^{t}*Bu zugegeben und die Suspension auf 80°C Innentemperatur erhitzt. Bei dieser Temperatur werden 25,9 ml (416 mmol) Methyliodid langsam zugetropft (1 Stunde, Temperatur < 90°C). Die Innentemperatur wird noch für weitere 1,5 Stunden bei 80 bis 90°C gehalten. Der abgekühlte Ansatz wird auf 1000 ml Eiswasser gegossen und 20 Minuten gerührt, der entstandene Feststoff abgesaugt, mit Wasser und MeOH gewaschen und sechsmal aus Toluol umkristallisiert. Man erhält einen hellgelben Feststoff mit einer Reinheit von > 99,9% in einer Ausbeute von 77% (21,4 g).

### Beispiel 2: Synthese von weiteren Monomeren

Die Synthese der im Folgenden beschriebenen, weiteren Monomere M2 bis M5 wird in der WO 03/020790 sowie der darin zitierten Literatur beschrieben.

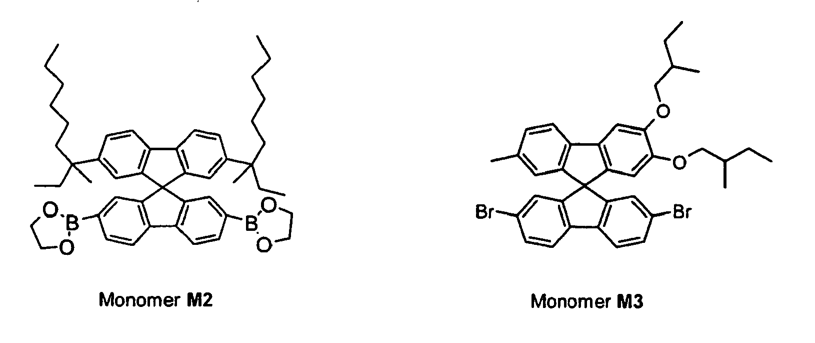

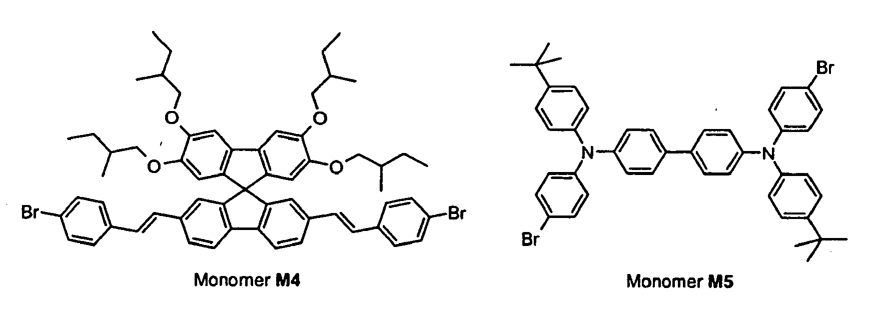

### Beispiel 3: Synthese der Polymere

Die erfindungsgemäßen Polymere sowie die Vergleichspolymere werden durch SUZUKI-Kupplung gemäß der WO 03/048225 synthetisiert. Die Zusammensetzung der synthetisierten, erfindungsgemäßen Polymere P1 und P2 sowie der Vergleichspolymere V1 und V2 ist in Tabelle 1 angegeben. Die Vergleichspolymere V1 und V2 weisen statt des Monomers M1, das im Polymer zu Einheiten gemäß Formel (1) führt, andere Monomere auf.

### Beispiel 4: Herstellung der PLEDs

Die Polymere werden für einen Einsatz in PLEDs untersucht. Die PLEDs sind jeweils Zweischichtsysteme, d.h. Substrat//ITO//PEDOT//Polymer// Kathode. PEDOT ist ein Polythiophen-Derivat (Baytron P, von H.C. Starck, Goslar). Als Kathode wird in allen Fällen Ba/Ag (Aldrich) verwendet. Wie PLEDs dargestellt werden, ist in der WO 04/037887 sowie der darin zitierten Literatur ausführlich beschrieben.

### Beispiele 5 bis 8: Device-Beispiele

Die Ergebnisse, die bei Verwendung der erfindungsgemäßen Polymere P1 und P2 in PLEDs erhalten werden, sind ebenfalls in Tabelle 1 zusammengefasst. Ebenso aufgeführt sind die Elektrolumineszenz-Ergebnisse, die unter Verwendung der Vergleichspolymere V1 und V2 erhalten werden.

Wie man aus den Ergebnissen erkennen kann, ist die Effizienz der erfindungsgemäßen Polymere besser als die der Vergleichspolymere. Die Emissionsfarbe ist vergleichbar und die Lebensdauern sind dies berücksichtigend deutlich verbessert. Dies zeigt, dass die erfindungsgemäßen Polymere besser für den Einsatz in Displays geeignet sind als Polymere gemäß dem Stand der Technik.

**Tabelle 1:**

| Beispiel | Polymer | Monomere | Max.Eff./ cd/A | U@100 cd/m² / V | CIE x/y^{a} | Lebensdauer^{b}/ h |
|---|---|---|---|---|---|---|
| 5 | P1 | 10% M1 | 7,7 | 2,8 | 0,16/0,24 | 400 |
| | | 50% M2 | | | | |
| | | 20% M3 | | | | |
| | | 10% M4 | | | | |
| | | 10% M5 | | | | |
| 6 | P2 | 2% M1 | 6,02 | 3,3 | 0,15/0,19 | 1200 |
| | | 50% M2 | | | | |
| | | 46% M3 | | | | |
| | | 2%M5 | | | | |
| 7 | V1 | 50% M2 | 4,66 | 3,9 | 0,17/0,30 | 180 |
| | | 30% M3 | | | | |
| | | 10% M4 | | | | |
| | | 10% M5 | | | | |
| 8 | V2 | 50% M2 | 4,70 | 4,7 | 0,17/0,26 | 449 |
| | | 46% M3 | | | | |
| | | 2%M4 | | | | |
| | | 2%M5 | | | | |

| | | | | | | |
|---|---|---|---|---|---|---|
| ^{a} CIE-Koordinaten: Farbkoordinaten der Commision Internationale de l'Eclairage 1931 ^{b} Lebensdauer: Zeit bis zum Abfall der Helligkeit auf 50 % der Anfangshelligkeit, Anfangshelligkeit 400 cd/m² | | | | | | |

## Patentansprüche

1. Konjugierte oder teilkonjugierte Polymere, enthaltend Einheiten der Formel (1), 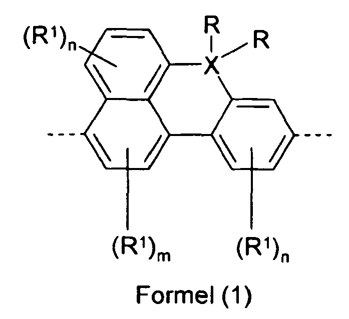 worin
R bei jedem Auftreten gleich oder verschieden H, eine geradkettige Alkylkette mit 1 bis 40 C-Atomen, eine verzweigte Alkylkette mit 3 bis 40 C-Atomen oder cyclische Alkylkette mit 3 bis 40 C-Atomen, die jeweils durch R¹ substituiert sein können, in der auch ein oder mehrere nicht benachbarte C-Atome durch =N-R¹, -O-, -S-, -O-CO-O-, -CO-O-, -CR¹=CR¹- oder -C≡C- ersetzt sein können und in der auch ein oder mehrere H-Atome durch F, Cl, Br, I oder CN ersetzt sein können, oder ein aromatisches Ringsystem mit 5 bis 40 C-Atomen oder hetero- aromatisches Ringsystem mit 2 bis 40 C-Atomen ist, welches auch durch ein oder mehrere Reste R¹ substituiert sein kann; dabei können die beiden Reste R miteinander auch ein weiteres mono- oder polycyclisches, aromatisches oder aliphatisches Ringsystem bilden; vorzugsweise mit der Maßgabe, dass mindestens einer oder beide Reste R ungleich H ist;
R¹ bei jedem Auftreten gleich oder verschieden H, eine geradkettige Alkylkette mit 1 bis 22 C-Atomen, eine verzweigte Alkylkette mit 3 bis 22 C-Atomen oder cyclische Alkylkette mit 3 bis 22 C-Atomen, in der auch ein oder mehrere nicht benachbarte C-Atome durch =N-R², -O-, -S-, -O-CO-O-, -CO-O-, -CR²=CR²- oder -C≡C- ersetzt sein können und in der auch ein oder mehrere H-Atome durch F, Cl, Br, I oder CN ersetzt sein können, oder eine Aryl-, Heteroaryl-, Aryloxy- oder Heteroaryloxygruppe mit 5 bis 40 C-Atomen ist, welche auch durch ein oder mehrere nicht-aromatische Reste R¹ substituiert sein kann; dabei können auch zwei oder mehrere der Reste R¹ miteinander und/oder mit R ein Ringsystem bilden; oder F, Cl, Br, I, CN, N(R²)₂, Si(R²)₃ oder B(R²)₂ bedeuten;
R² bei jedem Auftreten gleich oder verschieden H oder ein aliphatischer oder aromatischer Kohlenwasserstoffrest mit 1 bis 20 C-Atomen ist;
X bei jedem Auftreten C ist;
n bei jedem Auftreten gleich oder verschieden 0, 1, 2 oder 3 ist;
m bei jedem Auftreten gleich oder verschieden 0, 1 oder 2 ist; und
die gestrichelte Bindung dabei die Verknüpfung im Polymer bedeutet.

2. Polymere nach Anspruch 1, **dadurch gekennzeichnet, dass** sie neben den Einheiten der Formel (1) noch weitere Strukturelemente enthalten.

3. Polymere nach Anspruch 2, **dadurch gekennzeichnet, dass** die weiteren Strukturelemente, die die Lochinjektions- und/oder -transporteigenschaften erhöhen, ausgewählt sind aus den Gruppen der Triarylamin-, Benzidin-, Tetraaryl-para-phenylendiamin-, Triarylphosphin-, Phenothiazin-, Phenoxazin-, Dihydrophenazin-, Thianthren-, Dibenzo-para-dioxin-, Phenoxathiin-, Carbazol-, Azulen-, Thiophen-, Pyrrol- und Furanderivate und weiterer O-, S- oder N-haltiger Heterocyclen mit hoch liegendem HOMO.

4. Polymere nach mindestens einem der Ansprüche 2 oder 3, **dadurch gekennzeichnet, dass** die weiteren Strukturelemente, die die Elektroneninjektions- und/oder -transporteigenschaften erhöhen, ausgewählt sind aus den Gruppen der Pyridin-, Pyrimidin-, Pyridazin-, Pyrazin-, Oxadiazol-, Chinolin-, Chinoxalin- und Phenazinderivate, aber auch Triarylborane und weiterer O-, S- oder N-haltiger Heterocyclen mit niedrig liegendem LUMO.

5. Polymere nach mindestens einem der Ansprüche 2 bis 4, **dadurch gekennzeichnet, dass** die weiteren Strukturelemente Kombinationen von Einzeleinheiten gemäß Anspruch 3 und 4 aufweisen.

6. Polymere nach mindestens einem der Ansprüche 2 bis 5, **dadurch gekennzeichnet, dass** die weiteren Strukturelemente die die Emissionscharakteristik insoweit verändern, dass Elektrophosphoreszenz statt Elektrofluoreszenz erhalten werden kann, ausgewählt sind aus Verbindungen, welche Schweratome mit einer Ordnungszahl von mehr als 36 enthalten.

7. Polymere nach mindestens einem der Ansprüche 2 bis 6, **dadurch gekennzeichnet, dass** die weiteren Strukturelemente, die den Übergang vom Singulett- zum Triplettzustand verbessern, ausgewählt sind aus den Klassen der Carbazol- und überbrückten Carbazoldimereinheiten, Ketone, Phosphinoxide, Sulfoxide, Sulfone und Silan-Derivate.

8. Polymere nach mindestens einem der Ansprüche 2 bis 7, **dadurch gekennzeichnet, dass** die weiteren Strukturelementen, die die Morphologie und/oder die Emissionsfarbe der Polymere beeinflussen, ausgewählt sind aus den Klassen der 1,4-Phenylen-, 1,4-Naphthylen-, 1,4- oder 9,10-Anthrylen-, 1,6-, 2,7- oder 4,9-Pyrenylen-, 3,9- oder 3,10- Perylenylen-, 4,4'-Biphenylylen-, 4,4"-Terphenylylen-, 4,4'-Bi-1,1'-naphthylylen-, 4,4'-Tolanylen-, 4,4'-Stilbenylen- oder 4,4"-Bisstyrylarylenderivate.

9. Polymere nach mindestens einem der Ansprüche 2 bis 8, **dadurch gekennzeichnet, dass** die weiteren Strukturelementen, die typischerweise als Backbone verwendet werden, ausgewählt sind aus den Klassen der 4,5-Dihydropyrenderivate, 4,5,9,10-Tetrahydropyrenderivate, Fluorenderivate, 9,9'-Spirobifluorenderivate, 9,10-Dihydrophenanthrenderivate, 5,7-Dihydrodibenzooxepinderivate und cis- und trans-Indenofluorenderivate.

10. Polymere nach mindestens einem der Ansprüche 1 bis 9, **dadurch gekennzeichnet, dass** der Anteil der Einheiten der Formel (1) mindestens 5 mol% beträgt.

11. Polymere nach mindestens einem der Ansprüche 1 bis 10, **dadurch gekennzeichnet, dass** in Formel (1)
R bei jedem Auftreten gleich oder verschieden ist und für eine geradkettige Alkylkette mit 1 bis 25 C-Atomen, eine verzweigte Alkylkette mit 3 bis 25 C-Atomen oder cyclische Alkylkette mit 3 bis 25 C-Atomen, in der auch ein oder mehrere nicht benachbarte C-Atome durch =N-R¹, -O-, -S-, -O-CO-O-, -CO-O-, -CH=CH- oder -C≡C- ersetzt sein können und in der auch ein oder mehrere H-Atome durch F oder CN ersetzt sein können, oder eine aromatische oder hetero-aromatische Gruppe mit 4 bis 20 C-Atomen ist, welche auch durch ein oder mehrere nicht- aromatische Reste R¹ substituiert sein kann; dabei können die beiden Reste R miteinander auch ein weiteres mono- oder polycyclisches, aromatisches oder aliphatisches Ringsystem bilden; steht
R¹ bei jedem Auftreten gleich oder verschieden ist und für H, eine geradkettige Alkylkette mit 1 bis 22 C-Atomen, eine verzweigte Alkylkette mit 3 bis 22 C-Atomen oder cyclische Alkylkette mit 3 bis 22 C-Atomen, in der auch ein oder mehrere nicht benachbarte C-Atome durch =N-R², -O-, -S-, -O-CO-O-, -CO-O-, -CH=CH- oder -C≡C- ersetzt sein können und in der auch ein oder mehrere H-Atome durch F oder CN ersetzt sein können, oder eine Aryl-, Heteroaryl-, Aryloxy- oder Heteroaryloxygruppe mit 5 bis 40 C-Atomen ist, welche auch durch ein oder mehrere nicht-aromatische Reste R¹ substituiert sein kann; dabei können auch zwei oder mehrere der Reste R¹ miteinander und/oder mit R ein mono- oder polycyclisches, aliphatisches oder aromatisches Ringsystem bilden; oder F, Cl, Br, I, CN, N(R²)₂, Si(R²)₃ oder B(R²)₂ bedeuten, steht; und
X bei jedem Auftreten für C steht.

12. Mischungen (Blends) aus einem oder mehreren Polymeren nach mindestens einem der Ansprüche 1 bis 11 mit weiteren polymeren, oligomeren, dendritischen und/oder niedermolekularen Substanzen.

13. Lösungen und Formulierungen aus einem oder mehreren Polymeren nach mindestens einem der Ansprüche 1 bis 11 oder aus Blends nach Anspruch 12 in einem oder mehreren Lösungsmitteln.

14. Verwendung eines Polymers nach mindestens einem der Ansprüche 1 bis 11, eines Blends nach Anspruch 12 oder einer Lösung nach Anspruch 13 in Leuchtdioden.

15. Organisches elektronisches Bauteil mit einer oder mehreren aktiven Schichten, **dadurch gekennzeichnet, dass** mindestens eine dieser aktiven Schichten ein oder mehrere Polymere nach mindestens einem der Ansprüche 1 bis 11 oder Blends nach Anspruch 12 enthält.

16. Organisches elektronisches Bauteil nach Anspruch 15, **dadurch gekennzeichnet, dass** es sich um polymere Leuchtdioden (PLED), organische integrierte Schaltungen (O-IC), organische Feld-Effekt-Transistoren (OFET), organische Dünnfilmtransistoren (OTFT), organische Solarzellen (O-SC) oder organische Laserdioden (O-Laser) handelt.

17. Organisches elektronisches Bauteil nach Anspruch 16, **dadurch gekennzeichnet, dass** es sich um eine polymere Leuchtdiode handelt.

## Claims

1. Conjugated or partially conjugated polymers comprising units of the formula (1) 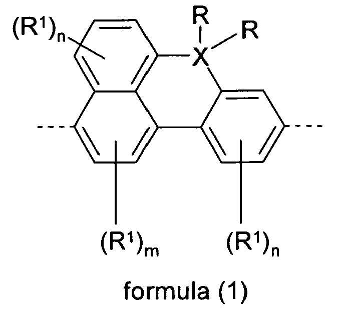 in which
R is on each occurrence, identically or differently, H, a straight- chain alkyl chain having 1 to 40 C atoms, a branched alkyl chain having 3 to 40 C atoms or a cyclic alkyl chain having 3 to 40 C atoms, each of which may be substituted by R¹ and in which, in addition, one or more non-adjacent C atoms may be replaced by =N-R¹ -O-, -S-, -O-CO-O-, -CO-O-, -CR¹=CR¹- or -C≡C- and in which, in addition, one or more H atoms may be replaced by F, Cl, Br, I or CN, or an aromatic ring system having 5 to 40 C atoms or a heteroaromatic ring system having 2 to 40 C atoms, which may also be substituted by one or more radicals R¹; the two radicals R here may also form a further mono- or polycyclic, aromatic or aliphatic ring system with one another; preferably with the proviso that at least one or both of the radicals R is (are) not equal to H;
R¹ is on each occurrence, identically or differently, H, a straight- chain alkyl chain having 1 to 22 C atoms, a branched alkyl chain having 3 to 22 C atoms or a cyclic alkyl chain having 3 to 22 C atoms, in which, in addition, one or more non-adjacent C atoms may be replaced by =N-R², -O-, -S-, -O-CO-O-, -CO-O-, -CR²=CR²- or -C≡C- and in which, in addition, one or more H atoms may be replaced by F, Cl, Br, I or CN, or an aryl, hetero- aryl, aryloxy or heteroaryloxy group having 5 to 40 C atoms, which may also be substituted by one or more non-aromatic radicals R¹; two or more of the radicals R¹ here may also form a ring system with one another and/or with R; or F, Cl, Br, I, CN, N(R²)₂, Si(R²)₃ or B(R²)₂;
R² is on each occurrence, identically or differently, H or an aliphatic or aromatic hydrocarbon radical having 1 to 20 C atoms;
X is on each occurrence C;
n is on each occurrence, identically or differently, 0, 1, 2 or 3;
m is on each occurrence, identically or differently, 0, 1 or 2; and
the dashed bond here denotes the linking in the polymer.

2. Polymers according to Claim 1, **characterised in that** they comprise further structural elements besides the units of the formula (1).

3. Polymers according to Claim 2, **characterised in that** the further structural elements which enhance the hole-injection and/or -transport properties are selected from the groups of the triarylamine, benzidine, tetraaryl-para-phenylenediamine, triarylphosphine, phenothiazine, phenoxazine, dihydrophenazine, thianthrene, dibenzo-para-dioxin, phenoxathiyne, carbazole, azulene, thiophene, pyrrole and furan derivatives and further O-, S- or N-containing heterocycles having a high HOMO.

4. Polymers according to at least one of Claims 2 or 3, **characterised in that** the further structural elements which enhance the electron-injection and/or -transport properties are selected from the groups of the pyridine, pyrimidine, pyridazine, pyrazine, oxadiazole, quinoline, quinoxaline and phenazine derivatives, but also triarylboranes and further O-, S- or N-containing heterocycles having a low LUMO.

5. Polymers according to at least one of Claims 2 to 4, **characterised in that** the further structural elements have combinations of individual units according to Claims 3 and 4.

6. Polymers according to at least one of Claims 2 to 5, **characterised in that** the further structural elements which modify the emission characteristics to such an extent that electrophosphorescence can be obtained instead of electrofluorescence are selected from compounds which contain heavy atoms having an atomic number greater than 36.

7. Polymers according to at least one of Claims 2 to 6, **characterised in that** the further structural elements which improve the transition from the singlet state to the triplet state are selected from the classes of the carbazole and bridged carbazole dimer units, ketones, phosphine oxides, sulfoxides, sulfones and silane derivatives.

8. Polymers according to at least one of Claims 2 to 7, **characterised in that** the further structural elements which influence the morphology and/or the emission colour of the polymers are selected from the classes of the 1,4-phenylene, 1,4-naphthylene, 1,4- or 9,10-anthrylene, 1,6-, 2,7- or 4,9-pyrenylene, 3,9- or 3,10-perylenylene, 4,4'-biphenylylene, 4,4"-terphenylylene, 4,4'-bi-1,1'-naphthylylene, 4,4'-tolanylene, 4,4'-stilbenylene and 4,4"-bisstyrylarylene derivatives.

9. Polymers according to at least one of Claims 2 to 8, **characterised in that** the further structural elements which are typically used as backbone are selected from the classes of the 4,5-dihydropyrene derivatives, 4,5,9,10-tetrahydropyrene derivatives, fluorene derivatives, 9,9'-spirobifluorene derivatives, 9,10-dihydrophenanthrene derivatives, 5,7-dihydrodibenzooxepine derivatives and cis- and trans-indenofluorene derivatives.

10. Polymers according to at least one of Claims 1 to 9, **characterised in that** the proportion of the units of the formula (1) is at least 5 mol%.

11. Polymers according to at least one of Claims 1 to 10, **characterised in that**, in formula (1),
R is on each occurrence identical or different and stands for a straight-chain alkyl chain having 1 to 25 C atoms, a branched alkyl chain having 3 to 25 C atoms or a cyclic alkyl chain having 3 to 25 C atoms, in which, in addition, one or more non-adjacent C atoms may be replaced by =N-R¹, -O-, -S-, -O-CO-O-, -CO-O-, -CH=CH- or -C≡C- and in which, in addition, one or more H atoms may be replaced by F or CN, or an aromatic or heteroaromatic group having 4 to 20 C atoms, which may also be substituted by one or more non-aromatic radicals R¹; the two radicals R here may also form a further mono- or polycyclic, aromatic or aliphatic ring system with one another;
R¹ is on each occurrence identical or different and stands for H, a straight-chain alkyl chain having 1 to 22 C atoms, a branched alkyl chain having 3 to 22 C atoms or a cyclic alkyl chain having 3 to 22 C atoms, in which, in addition, one or more non-adjacent C atoms may be replaced by =N-R², -O-, -S-, -O-CO-O-, -CO-O-, -CH=CH- or -C≡C- and in which, in addition, one or more H atoms may be replaced by F or CN, or an aryl, hetero- aryl, aryloxy or heteroaryloxy group having 5 to 40 C atoms, which may also be substituted by one or more non-aromatic radicals R¹; two or more of the radicals R¹ here may also form a mono- or polycyclic, aliphatic or aromatic ring system with one another and/or with R; or F, Cl, Br, I, CN, N(R²)₂, Si(R²)₃ or B(R²)₂; and
X on each occurrence stands for C.

12. Mixtures (blends) of one or more polymers according to at least one of Claims 1 to 11 with further polymeric, oligomeric, dendritic and/or low-molecular-weight substances.

13. Solutions and formulations comprising one or more polymers according to at least one of Claims 1 to 11 or blends according to Claim 12 in one or more solvents.

14. Use of a polymer according to at least one of Claims 1 to 11, a blend according to Claim 12 or a solution according to Claim 13 in light-emitting diodes.

15. Organic electronic component having one or more active layers, **characterised in that** at least one of these active layers comprises one or more polymers according to at least one of Claims 1 to 11 or blends according to Claim 12.

16. Organic electronic component according to Claim 15, **characterised in that** it is a polymeric light-emitting diode (PLED), organic integrated circuit (O-IC), organic field-effect transistor (O-FET), organic thin-film transistor (O-TFT), organic solar cell (O-SC) or organic laser diode (O-laser).

17. Organic electronic component according to Claim 16, **characterised in that** it is a polymeric light-emitting diode.

## Revendications

1. Polymères conjugués ou partiellement conjugués comprenant des unités de la formule (1) 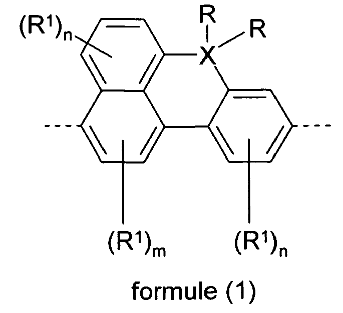 dans laquelle
R est, pour chaque occurrence, de manière identique ou diffé- rente, H, une chaîne alkyle en chaîne droite comportant de 1 à 40 atomes de C, une chaîne alkyle ramifiée comportant de 3 à 40 atomes de C ou une chaîne alkyle cyclique comportant de 3 à 40 atomes de C, dont chacune peut être substituée par R¹ et où, en plus, un ou plusieurs atomes de C non adjacents peut/ peuvent être remplacé(s) par =N-R¹, -O-, -S-, -O-CO-O-, -CO-O-, -CR¹=CR¹- ou -C≡C- et où, en plus, un ou plusieurs atomes de H peut/peuvent être remplacé(s) par F, Cl, Br, I ou CN, ou un système de cycle aromatique comportant de 5 à 40 atomes de C ou un système de cycle hétéroaromatique com- portant de 2 à 40 atomes de C, lequel peut également être substitué par un ou plusieurs radicaux R¹ ; les deux radicaux R afférents peuvent également former un autre système de cycle aromatique ou aliphatique, mono- ou polycyclique l'un avec l'autre ; de préférence étant entendu qu'au moins l'un des radi- caux R ou les deux est/sont non égal/égaux à H ;
R¹ est, pour chaque occurrence, de manière identique ou diffé- rente, H, une chaîne alkyle en chaîne droite comportant de 1 à 22 atomes de C, une chaîne alkyle ramifiée comportant de 3 à 22 atomes de C ou une chaîne alkyle cyclique comportant de 3 à 22 atomes de C, où, en plus, un ou plusieurs atomes de C non adjacents peut/peuvent être remplacé(s) par =N-R², -O-, -S-, -O-CO-O-, -CO-O-, -CR²=CR²- ou -C≡C- et où, en plus, un ou plusieurs atomes de H peut/peuvent être remplacé(s) par F, Cl, Br, I ou CN, ou un groupe aryle, hétéroaryle, aryloxy ou hétéroaryloxy comportant de 5 à 40 atomes de C, lequel peut également être substitué par un ou plusieurs radicaux non aro- matiques R¹ ; deux des radicaux R¹ afférents ou plus peuvent également former un système de cycle les uns avec les autres et/ou avec R ; ou F, Cl, Br, I, CN, N(R²)₂, Si(R²)₃ ou B(R²)₂ ;
R² est, pour chaque occurrence, de manière identique ou diffé- rente, H ou un radical hydrocarbone aliphatique ou aromatique comportant de 1 à 20 atomes de C ;
X est, pour chaque occurrence, C ;
n est, pour chaque occurrence, de manière identique ou diffé- rente, 0, 1, 2 ou 3 ;
m est, pour chaque occurrence, de manière identique ou diffé- rente, 0, 1 ou 2 ; et
la liaison en pointillés représente ici la liaison dans le polymère.

2. Polymères selon la revendication 1, **caractérisés en ce qu'**ils comprennent d'autres éléments structurels en plus des unités de la formule (1).

3. Polymères selon la revendication 2, **caractérisés en ce que** les autres éléments structurels, lesquels améliorent les propriétés d'injection et/ou de transport de trous, sont choisis parmi les groupes constitués par des dérivés de triarylamine, benzidine, tétraaryl-para-phénylènediamine, triarylphosphine, phénothiazine, phénoxazine, dihydrophénazine, thianthrène, dibenzoparadioxine, phénoxathiyne, carbazole, azulène, thiophène, pyrrole et furane et en outre, des hétérocycles contenant O, S ou N présentant un HOMO élevé.

4. Polymères selon au moins l'une des revendications 2 ou 3, **caractérisés en ce que** les autres éléments structurels, lesquels améliorent les propriétés d'injection et/ou de transport d'électrons, sont choisis parmi les groupes constitués par des dérivés de pyridine, pyrimidine, pyridazine, pyrazine, oxadiazole, quinoline, quinoxaline et phénazine, mais également des triarylboranes et en outre, des hétérocycles contenant O, S ou N présentant un LUMO faible.

5. Polymères selon au moins l'une des revendications 2 à 4, **caractérisés en ce que** les autres éléments structurels comportent des combinaisons d'unités individuelles selon les revendications 3 et 4.

6. Polymères selon au moins l'une des revendications 2 à 5, **caractérisés en ce que** les autres éléments structurels, lesquels modifient les caractéristiques d'émission selon un degré tel qu'une électrophosphorescence peut être obtenue en lieu et place d'une électrofluorescence, sont choisis parmi des composés qui contiennent des atomes lourds présentant un numéro atomique supérieur à 36.

7. Polymères selon au moins l'une des revendications 2 à 6, **caractérisés en ce que** les autres éléments structurels, lesquels améliorent la transition de l'état de singlet à l'état de triplet, sont choisis parmi les classes des unités de dimère de carbazole et de carbazole ponté, les cétones, les oxydes de phosphine, les sulfoxydes, les sulfones et les dérivés de silane.

8. Polymères selon au moins l'une des revendications 2 à 7, **caractérisés en ce que** les autres éléments structurels, lesquels influencent la morphologie et/ou les couleurs d'émission des polymères, sont choisis parmi les classes des dérivés de 1,4-phénylène, 1,4-naphthylène, 1,4- ou 9,10-anthrylène, 1,6-, 2,7- ou 4,9-pyrénylène, 3,9- ou 3,10-pérylènylène, 4,4'-biphénylylène, 4,4"-terphénylylène, 4,4'-bi-1,1'-naphthylylène, 4,4'-tolanylène, 4,4'-stilbénylène et 4,4"-bisstyrylarylène.

9. Polymères selon au moins l'une des revendications 2 à 8, **caractérisés en ce que** les autres éléments structurels, lesquels sont typiquement utilisés en tant qu'ossature, sont choisis parmi les classes des dérivés de 4,5-dihydropyrène, des dérivés de 4,5,9,10-tétrahydropyrène, des dérivés de fluorène, des dérivés de 9,9'-spirobifluorène, des dérivés de 9,10-dihydrophénanthrène, des dérivés de 5,7-dihydrodibenzooxépine et des dérivés de cis- et trans-indénofluorène.

10. Polymères selon au moins l'une des revendications 1 à 9, **caractérisés en ce que** la proportion des unités de la formule (1) est d'au moins 5 mol%.

11. Polymères selon au moins l'une des revendications 1 à 10, **caractérisés en ce que**, dans la formule (1),
R est pour chaque occurrence identique ou différent et représente une chaîne alkyle en chaîne droite comportant de 1 à 25 atomes de C, une chaîne alkyle ramifiée comportant de 3 à 25 atomes de C ou une chaîne alkyle cyclique comportant de 3 à 25 atomes de C, où, en plus, un ou plusieurs atomes de C non adjacents peut/peuvent être remplacé(s) par =N-R¹, -O-, -S-, -O-CO-O-, -CO-O-, -CH=CH- ou -C≡C- et où, en plus, un ou plusieurs atomes de H peut/peuvent être remplacé(s) par F ou CN, ou un groupe aromatique ou hétéroaromatique comportant de 4 à 20 atomes de C, lequel peut également être substitué par un ou plusieurs radicaux R¹ non aromatiques ; les deux radicaux R afférents peuvent également former un autre sys- tème de cycles aromatique ou aliphatique, mono- ou poly- cyclique l'un avec l'autre ;
R¹ est pour chaque occurrence identique ou différent et représente H, une chaîne alkyle en chaîne droite comportant de 1 à 22 atomes de C, une chaîne alkyle ramifiée comportant de 3 à 22 atomes de C ou une chaîne alkyle cyclique comportant de 3 à 22 atomes de C, où, en outre, un ou plusieurs atomes de C non adjacents peut/peuvent être remplacé(s) par =N-R², -O-, -S-, -O-CO-O-, -CO-O-, -CH=CH- ou -C≡C- et où, en plus, un ou plusieurs atomes de H peut/peuvent être remplacé(s) par F ou CN, ou un groupe aryle, hétéroaryle, aryloxy ou hétéroaryloxy comportant de 5 à 40 atomes de C, lequel peut également être substitué par un ou plusieurs radicaux R¹ non aromatiques ; deux des radicaux R¹ afférents ou plus peuvent également for- mer un système de cycle aliphatique ou aromatique mono- ou polycyclique les uns avec les autres et/ou avec R ; ou F, Cl, Br, I, CN, N(R²)₂, Si(R²)₃ ou B(R²)₂ ; et
X pour chaque occurrence représente C.

12. Mélanges (blends) d'un ou de plusieurs polymères selon au moins l'une des revendications 1 à 11 avec d'autres substances polymériques, oligomériques, dendritiques et/ou de poids moléculaire faible.

13. Solutions et formulations comprenant un ou plusieurs polymères selon au moins l'une des revendications 1 à 11 ou blends selon la revendication 12 dans un ou plusieurs solvants.

14. Utilisation d'un polymère selon au moins l'une des revendications 1 à 11, d'un blend selon la revendication 12 ou d'une solution selon la revendication 13 dans des diodes émettrices de lumière.

15. Composant électronique organique comportant une ou plusieurs couches actives, **caractérisé en ce qu'**au moins l'une de ces couches actives comprend un ou plusieurs polymères selon au moins l'une des revendications 1 à 11 ou des blends selon la revendication 12.

16. Composant électronique organique selon la revendication 15, **caractérisé en ce qu'**il s'agit d'une diode émettrice de lumière polymérique (PLED), d'un circuit intégré organique (O-IC), d'un transistor à effet de champ organique (O-FET), d'un transistor à film mince organique (O-TFT), d'une cellule solaire organique (O-SC) ou d'une diode laser organique (O-laser).

17. Composant électronique organique selon la revendication 16, **caractérisé en ce qu'**il s'agit d'une diode émettrice de lumière polymérique.
